# FASCICULE DE BREVET EUROPEEN

(11) **EP 2 869 774 B1**
(45) Date de publication et mention de la délivrance du brevet: **14.09.2016**
(21) Numéro de dépôt: 13747433.4
(22) Date de dépôt: 03.07.2013
(51) Int. Cl.: A61B 17/70, A61B 17/86

(54) **VIS POLYAXIALE A FILETAGE MÉCANIQUE ET SON DISPOSITIF DE FROTTEMENT**
POLYAXIALE SCHRAUBE MIT EINEM MECHANISCHEN GEWINDE UND REIBUNGSVORRICHTUNG DAFÜR
POLYAXIAL SCREW HAVING A MECHANICAL THREAD, AND FRICTION DEVICE THEREOF

(30) Priorité: 06.07.2012 FR 1256503
(43) Date de publication de la demande: 13.05.2015
(73) Titulaire: Clariance, 62000 Dainville (FR)
(72) Inventeur: TORNIER, Alain, F-38330 Saint Ismier (FR); VIART, Guy, F-62128 Saint Leger (FR); LEROY, Jean Yves, F-62870 Campagne-les-hesdin (FR); SAUVAGE, Bruno, F-62690 Frevin Capelle (FR)
(74) Mandataire: Godard, Xavier
(86) Numéro de dépôt international: PCT/FR2013/051581
(87) Numéro de publication internationale: WO 2014/006334

(56) Documents cités:
- WO-A1-96/29947
- WO-A1-2007/041686
- WO-A2-00/62692

## Description

La présente invention est relative à une vis poly axiale comportant entre sa partie filetée d'ancrage osseux et sa partie d'ancrage à filetage mécanique un dispositif de frottement et un dispositif de retenue assurant un pré-positionnement axiale desdites parties l'une par rapport à l'autre.

On connaît des vis poly axiales comportant une partie filetée d'ancrage osseux reliée à une partie d'ancrage à filetage mécanique qui est totalement libre en rotation autour de ladite partie filetée d'ancrage osseux.

Cette liberté de mouvement de la partie d'ancrage à filetage mécanique par rapport à la partie filetée d'ancrage osseux présente certains inconvénients lorsque ladite vis est ancrée dans le corps vertébral d'une vertèbre.

En effet la partie d'ancrage à filetage mécanique est libre de tout pivotement autour de la partie filetée d'ancrage osseux pendant la mise en place du connecteur de liaison et de la tige de liaison par le chirurgien.

Cette liberté de mouvement de la partie d'ancrage à filetage mécanique ne permet pas de positionner et de maintenir cette dernière dans un plan axial déterminé pour le montage du connecteur de liaison et de la tige de liaison par le chirurgien.

Ce dernier est dans l'obligation de maintenir la partie d'ancrage à filetage mécanique dans une position angulaire déterminée par rapport à la partie filetée d'ancrage osseux fixée dans le corps d'une vertébre pendant la mise en place du connecteur de liaison et de la tige de liaison entrainant une réelle complication de montage du dispositif rachidien.

On connait également des vis poly axiales exposées dans le document WO 2007/041686, qui comportent une bague de sécurité configurée pour former un dispositif de frottement d'encliquetage.

La vis poly axiale suivant la présente invention vise à remédier à ses inconvénients en proposant entre la partie d'ancrage à filetage mécanique et la partie filetée d'ancrage osseux des moyens de frottement et de retenue assurant le pré-positionnement axiale desdites parties d'ancrage.

La vis poly axiale suivant la présente invention comporte une partie fileté d'ancrage osseux et une partie d'ancrage à filetage mécanique pourvues respectivement d'une tête reliée entre elle par des moyens de retenue, des moyens de frottement disposés entre la tête de la partie filetée d'ancrage osseux et la tête de la partie d'ancrage à filetage mécanique, lesdits moyens de retenue assurant que les parties d'ancrage ne puissent se séparer l'une de l'autre tout en garantissant à la partie d'encrage à filetage mécanique des libertés de mouvement en translation vertical et en pivotement angulaire par rapport à ladite partie filetée d'ancrage osseux afin de pouvoir pré-positionner axialement avant son immobilisation ladite partie d'ancrage à filetage mécanique par rapport à la position d'ancrage axiale de la partie filetée d'ancrage osseux

La vis poly axiale suivant la présente invention comporte des moyens de frottement qui sont constitués d'un fil hélicoïdal en forme de ressort positionné à l'intérieur d'un logement ménagé dans la tête de la partie filetée d'ancrage osseux et sur lequel vient prendre appui la tête à profil externe sphérique de la partie d'ancrage à filetage mécanique.

La vis poly axiale suivant la présente invention comporte des moyens de retenue qui sont constitués de deux demi-coquilles logées à l'intérieur d'une rainure ménagée dans la tête de la partie filetée d'ancrage osseux.

La vis poly axiale suivant la présente invention comporte deux demi-coquilles dont le profil interne est de forme complémentaire à celui externe de la tête sphérique de la partie d'ancrage à filetage mécanique.

La vis poly axiale suivant la présente invention comporte une partie d'ancrage à filetage mécanique qui est sécable.

La vis poly axiale suivant la présente invention comporte une partie d'ancrage à filetage mécanique comprenant à l'opposé de la tête sphérique une empreinte pour l'introduction d'un outil.

La vis poly axiale suivant la présente invention comporte des moyens de frottement qui sont constitués d'un fil hélicoïdal en forme de ressort réalisé dans un matériau à fort coefficient de frottement tel que de l'alliage de titane ou de cobalt.

La vis poly axiale suivant la présente invention comporte un logement aménagé à l'intérieur de la tête de la partie filetée d'ancrage osseux qui présente un profil sphérique.

La vis poly axiale suivant la présente invention comporte une partie filetée d'ancrage osseux dont la tête présente sur sa face externe des empreintes permettant la mise en place d'un outil pour l'entrainement en rotation de ladite partie filetée d'ancrage osseux.

La vis poly axiale suivant la présente invention comporte une partie filetée d'ancrage osseux dont la tête présente sur sa face externe un filetage permettant la mise en place des moyens de retenue assurant que les parties d'ancrage ne puissent se séparer l'une de l'autre tout en garantissant à la partie d'ancrage à filetage mécanique des libertés de mouvement en translation vertical et. en pivotement angulaire par rapport à ladite partie filetée d'ancrage osseux.

La vis poly axiale suivant la présente invention comporte un logement aménagé à l'intérieur de la tête de la partie filetée d'ancrage osseux comprenant en son milieu une empreinte cylindrique qui est prévue pour recevoir l'une des extrémités du fil hélicoïdal en forme de ressort des moyens de frottement.

La vis poly axiale suivant la présente invention comporte une partie d'ancrage à filetage mécanique dont la tête comprend en son milieu une empreinte cylindrique qui est prévue pour recevoir l'une des extrémités du fil hélicoïdal en forme de ressort des moyens de frottement.

La vis poly axiale suivant la présente invention composte des moyens de retenue qui sont constitués d'un écrou comportant une face interne sphérique coopérant avec le profil externe sphérique de la tête de la partie d'ancrage à filetage mécanique et une face externe sphérique permettant la réception d'un connecteur de liaison d'un dispositif de fixation rachidien.

La description qui va suivre en regard des dessins annexés, donnés à titre d'exemples non limitatifs permettra de mieux comprendra l'invention, les caractéristiques qu'elle présente, et les avantages qu'elle est susceptible de procurer:
Figure 1 est une vue en perspective éclatée illustrant une vis poly axiale à filetage mécanique suivant la présente invention.
Figures 2 et 3 sont des vues en coupe montrant la vis poly axiale à filetage mécanique suivant la présente invention dans différentes postions axiales.
Figures 4 à 6 sont des vues représentant une variante de réalisation de la vis poly axiale à filetage mécanique suivant la présente invention.
Figure 7 est une vue illustrant un exemple dé montage de la vis poly axiale à filetage mécanique suivant la présente invention avec un connecteur

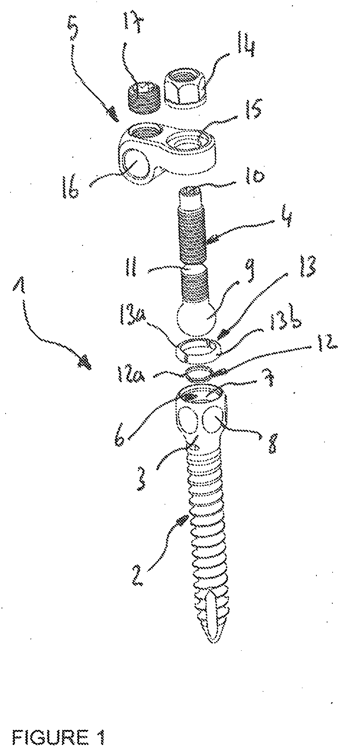

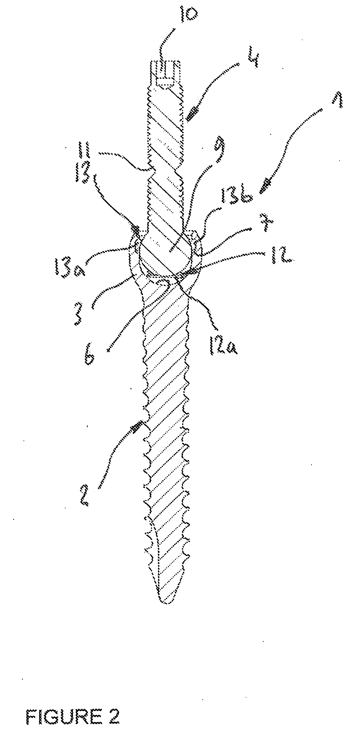

On a montré en figures 1 à 3 une vis poly axiale 1 comportant une partie fileté d'ancrage osseux 2 et une partie d'ancrage à filetage mécanique 4 pourvues respectivement d'une tête 3 et 9 reliées entre elles par des moyens de retenue 13 assurant à la partie d'ancrage à filetage mécanique 4 une position angulaire prédéterminée afin de pouvoir immobiliser sur cette dérnière un connecteur de liaison 5 d'un dispositif de fixation rachidien.

A l'intérieur de la tête 3 de la partie filetée d'ancrage osseux 2 est aménagé un logement interné 6 à un profil sphérique et une rainure périphérique 7.

La tête 3 présente sur sa face externe des empreintes 8 permettant la mise en place d'un outil, non illustré, pour l'entrainement en rotation de la partie filetée d'ancrage osseux 2 de la vis poly axiale 1.

La partie d'ancrage à filetage mécanique 4 est solidaire à l'une de ses extrémités d'une tête 9 à profil externe sphérique, tandis que extrémité opposée est percé en son milieu d'une empreinte 10 pour l'introduction d'un outil, non représenté.

La partie d'ancrage à filetage mécanique 4 de la vis poly axiale 1 comporte entre la tête sphérique 9 et son extrémité libre au moins une rainure 11 assurant une zone de rupture de ladite partie d'ancrage à filetage mécanique 4 afin que cette dernière soit sécable sous un effort de rotation prédéterminé.

La vis poly axiale 1 comporte des moyens de frottement 12 disposés à l'intérieur de la tête 3 de la partie filetée d'ancrage osseux 2 et sur laquelle vient prendre appui la tête sphérique 9 de la partie d'ancrage à filetage mécanique 4 afin de pouvoir pré-positionner axialement avant son immobilisation ladite partie d'ancrage à filetage mécanique 4 par rapport à la position d'ancrage axiale de la partie filetée d'ancrage osseux 2 de la vis poly-axiale 1 dans le corps vertébral d'une vertébre.

La vis poly axiale 1 comporte également des moyens de retenue 13 permettant d'empêcher la tête sphérique 9 de la partie d'ancrage à filetage mécanique 4 de sortir du logement interne 6 de la tête 3 de la partie filetée d'ancrage osseux 2 lorsque les deux parties d'ancrage 2 et 4 sont assemblées.

Les moyens de frottement 12 sont constitués d'un fil hélicoïdal 12a en forme de ressort positionné à l'intérieur de la tête 3 et plus particulièrement dans le fond du logement 6 de la partie filetée d'ancrage osseux 2 et sur lequel vient prendre appui la tête sphérique 9 de la partie d'ancrage à filetage mécanique 4.

Le fil hélicoïdal 12a peut être réalisé dans un matériau à fort coefficient de frottement tel que par exemple de l'alliage de titane ou de l'alliage de cobalt.

Les moyens de retenue 13 sont constitués de deux demi coquilles 13a et 13b logés dans la rainure 7 ménagée à l'intérieur du logement 6 de la tête 3 de la partie filetée d'ancrage osseux 2.

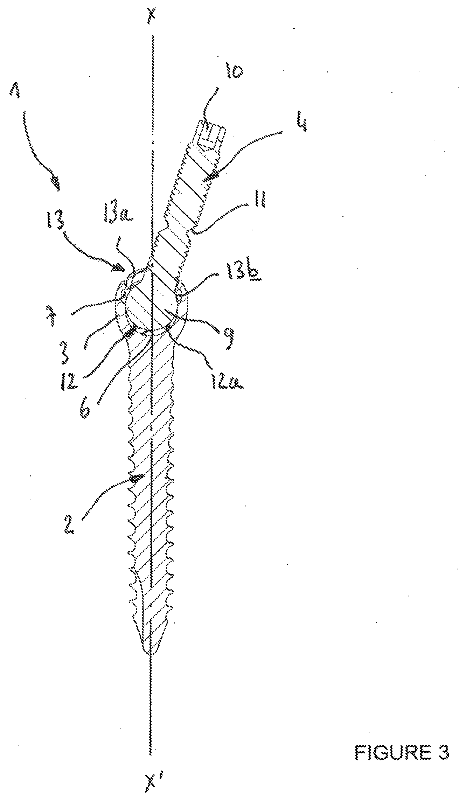

On a montré en figure 3 la mise en place de la demi coquille 13a à l'intérieur de la rainure 7 sachant que l'autre demi coquille 13b est introduite de la même manière.

En effet la demi coquille 13a est introduite à l'intérieur de la rainure 7 lorsque la partie d'ancrage à filetage mécanique 4 est positionnée suivant une direction inclinée dirigée vers la droite par rapport à l'axe principal et vertical xx' de la partie filetée d'ancrage osseux 2.

Versement, l'autre demi coquille 13b est introduite de la même manière lorsque la partie d'ancrage à filetage mécanique 4 est positionnée suivant une direction inclinée dirigée vers la gauche par rapport à l'axe principal et vertical xx' de la partie filetée d'ancrage osseux 2.

Chaque position inclinée à droite ou à gauche de la partie d'ancrage à filetage mécanique 4 par rapport à la partie filetée d'ancrage osseux 2 permet de libérer l'accès à la rainure 7 ménagée à l'intérieur du logement 6 de la tête 3 pour introduction de deux demi coquilles 13a et 13b.

Lorsque les deux demi coquilles 13a et 13b sont logés à l'intérieur de la rainure 7 ces derniéres permettent d'assurer que les parties d'ancrage 2 et 4 ne puassent se séparer tout en garantissant à la partie d'ancrage à filetage mécanique 4 des libertés de mouvement en translation vertical et en pivotement angulaire par rapport à la partie filetée d'ancrage osseux 2 implanté dans le corps vertébral, d'une vertébre.

Les moyens de frottement 12 constitués du fil hélicoïdal 12a en formé de ressort permettent de maintenir la tête sphérique 9 de la partie d'ancrage à filetage mécanique 4 contre les deux demi coquilles 13a et 13b des moyens de retenue 13 dont le profil interne est de forme complémentaire à celui externe de ladite tête sphérique 9.

Les moyens de frottement 12 permettent de maintenir dans une position axiale, déterminée par le chirurgien, la partie d'ancrage à filetage mécanique 4 par rapport à la partie filetée d'ancrage osseux 2 pendant le montage du connecter de liaison 5 et avant son immobilisation en rotation par l'intermédiaire d'un écrou de serrage 14.

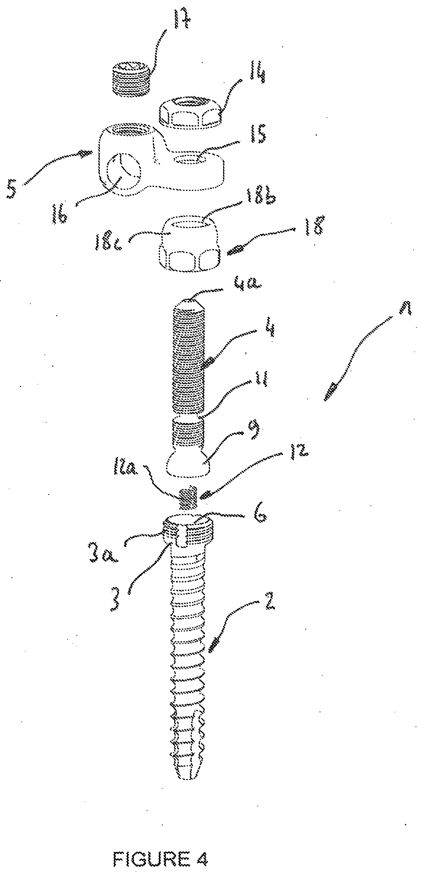

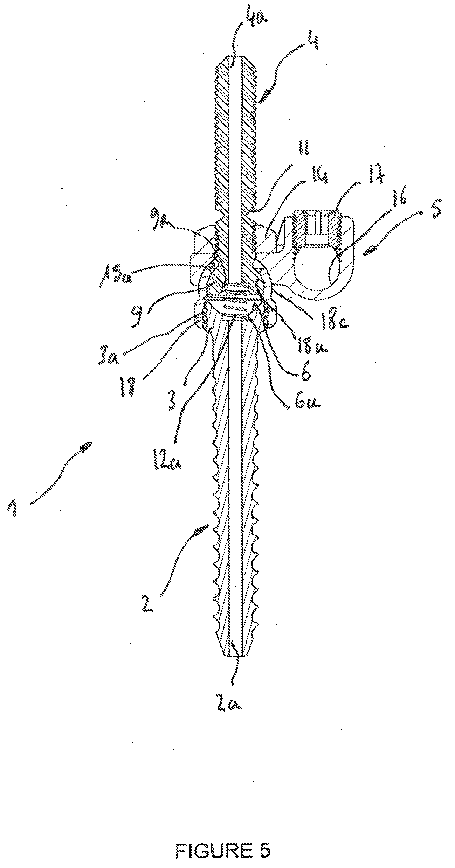

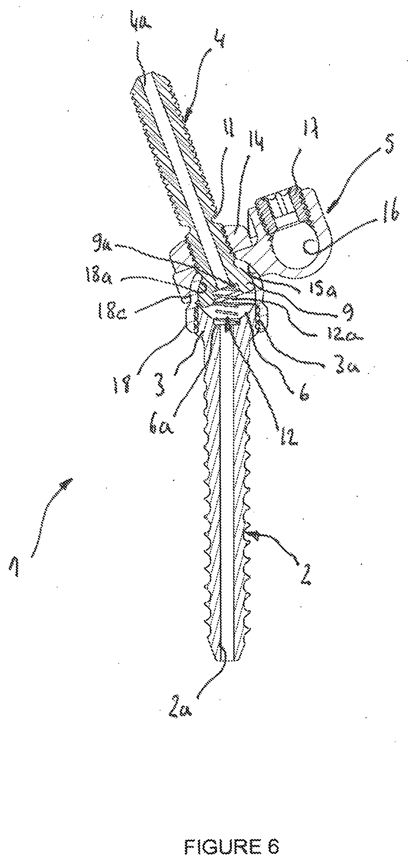

On a montré en figures 4 à 6 une variante de réalisation de la vis poly axiale 1 comportant une partie fileté d'ancrage osseux 2 et une partie d'ancrage à filetage mécanique 4 pourvue respectivement d'une tête 3 et 9 reliées entre elles par des moyens de retenue 18 assurant à ladite partie d'ancrage à filetage mécanique 4 une position angulaire prédéterminée afin de pourvoir immobiliser sur cette dernière un connecteur de liaison 5 d'un dispositif de fixation rachidien.

A l'intérieur de la tête 3 de la partie filetée d'ancrage osseux 2 est aménagé une logement interne 6 à un profil sphérique. Le corps de la partie filetée d'ancrage osseux 2 est percé en son milieu d'un alésage 2a débouchant à chaque extrémité de ladite partie.

L'alésage 2a débouche dans une empreinte cylindrique 6a ménagée dans le fond et au milieu du logement interne 6 et dont le diamètre interne est supérieure à celui dudit alésage.

L'empreinte cylindrique 6a est prévue pour recevoir l'une des extrémités du fil hélicoïdal 12a en forme de ressort des moyens de frottement 12.

La tête 3 de la partie filetée d'ancrage osseux 2 présente sur sa face externe un filetage 3a sur lequel sont vissés les moyens de retenue 18 permettant lors du montage de la vis poly axial 1 de réunir lesdites parties filetée d'ancrage osseux 2 et à filetage mécanique 4 entrent-elles.

Les moyens de retenue sont constitués d'un écrou de liaison 18 comportant une ouverture 18b qui est traversée par la partie d'ancrage à filetage mécanique 4 permettant à ladite partie d'ancrage à filetage mécanique 4 lorsque la vis poly axiale 1 est assemblée de pouvoir pivoter autour de l'axe principal et vertical xx'.

La partie d'ancrage à filetage mécanique 4 est pourvue à l'une de ses extrémités d'une tête 9 à profil externe sphérique. Le corps de la partie d'ancrage à filetage mécanique 4 est percé en son milieu d'un alésage 4a débouchant à chaque extrémité de ladite partie.

L'alésage 4a débouche dans une empreinte cylindrique 9a ménagée dans la tête 9 et dont le diamètre interne est supérieure à celui dudit alésage.

L'empreinte cylindrique 9a est prévue pour recevoir l'autre extrémité du fil hélicoïdal 12a en forme de ressort des moyens de frottement 12 lorsque les têtes 3 et 9 sont assemblées par l'intermédiaire de l'écrou de liaison 18.

En effet, l'écrou de liaison 18 comporte une face interne 18a dont le profil est de forme complémentaire à celui externe sphérique de la tête 9 de la partie d'ancrage à filetage mécanique 4.

L'assemblage de la vis poly axiale 1 et plus particulièrement de la partie filetée d'ancrage osseux 2 avec la partie d'ancrage à filetage mécanique 4 s'effectue de la manière suivante:
- l'écrou de liaison 18 est placé autour de la tête 9 de la partie d'ancrage à filetage mécanique 4 de manière que la face interne 18a soit en contact avec le profil sphérique externe de ladite tête 9;
- les moyens de frottement 12 et plus particulièrement les extrémités du fil hélicoïdal 12a en forme de ressort sont placés respectivement dans l'empreinte cylindrique 6a, 9a correspondante des têtes 3 et 9 lors du serrage de l'écrou de liaison 18 autour de ladite tête 3 de la partie fileté d'ancrage osseux partie d'ancrage osseux 2.

On note que le serrage complet de l'écrou de liaison 18 sur la tête 3 de la partie filetée d'ancrage osseux 2 permet de comprimer le fil hélicoïdale 12a en forme de ressort des moyens de frottement 12 entrainant une poussée de la tête 9 sur la face interne 18a dudit écrou de liaison 18.

Cet effort de poussé assure une contrainte de frottement de la tête 9 contre la face interne 18a de l'écrou de liaison 18 tout en garantissant à la partie d'ancrage à filetage mécanique 4 des libertés de mouvement en translation vertical et en pivotement angulaire par rapport à la partie filetée d'ancrage osseux 2 lorsqu'on est implanté dans le corps vertébral d'une vertébre.

Le diamètre externe sphérique de la tête 9 de la partie d'ancrage à filetage mécanique 4 est prévu de dimensions inférieures à celui interne du logement sphérique 6 de la partie filetée d'ancrage osseux 2 permettant un guidage lors des déplacements angulaire de la partie d'ancrage à filetage mécanique 4 par rapport à ladite partie filetée d'ancrage osseux 2 de la vis poly axiale 1.

On note que le connecteur de liaison 5 peut être constitué d'une part d'un premier alésage 15 traversé par la partie d'ancrage à filetage mécanique 4 de la vis poly-axiale 1 et d'autre part d'un second alésage 16 perpendiculaire au premier pour le passage d'une tige de liaison non représenté et qui est immobilisée en rotation et en translation à l'intérieur dudit second alésage 16 par une vis de pression 17.

L'écrou 18 comporte un profil externe 18c en portion de sphère destiné à coopérer avec un logement 15a de profil complémentaire aménagée à l'une des extrémité du premier alésage 15 du connecteur 5 et traversé par la partie d'ancrage à filetage mécanique 4 de la vis poly axiale 1.

Cet agencement permet d'assurer lors du réglage angulaire de la partie d'ancrage à filetage mécanique 4 par rapport à la partie filetée d'ancrage osseux 2 de guider axialement le connecteur de liaison 5 par rapport à la position angulaire de ladite partie d'ancrage à filetage mécanique 4.

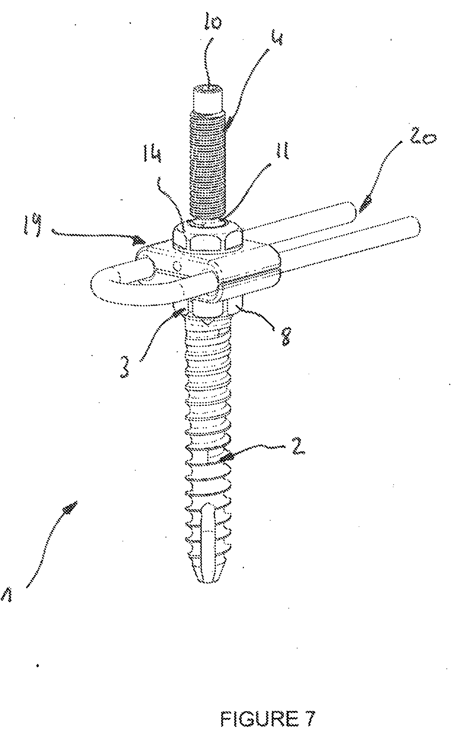

On a montré en figure 7 un autre exemple de montage de la vis poly-axiale 1 suivant la présente invention avec un connecteur 19 à double tige de liaison 20. Le connecteur 19 vient en lieu et place du connecteur 5 de manière que l'écrou de serrage 14 compère avec la partie d'ancrage à filetage mécanique 4.

Il doit d'ailleurs être entendu que la description qui précéde n'a été donnée qu'à titre d'exemple et quelle ne limite nullement le domaine de l'invention dont on ne sortirait pas en remplaçant les détails d'exécution décrits par tout autre équivalent.

## Revendications

1. Vis poly axiale comportant une partie filetée d'ancrage osseux (2) et une partie d'ancrage à filetage mécanique (4) pourvues respectivement d'une tête (3. 9) reliées entre elles par des moyens de retenue (13, 18), comportant d'une part des moyens de frottement (12) disposés entre la tête (3) de la partie d'ancrage osseux (2) et la tête (9) de la partie d'ancrage à filetage mécanique (4) **caractérisé en ce qu'**ils sont constitués d'un fil hélicoïdal (12a) en forme de ressort positionné à l'intérieur d'un logement (6) ménagé dans la tête (3) de la partie filetée d'ancrage osseux (2) et sur lequel vient prendre appui la tête (9) à profil externe sphérique de la partie d'ancrage à filetage mécanique (4) et d'autre part des moyens de retenue (13, 18) assurant que les parties d'ancrage (2, 4) ne puissent se séparer l'une de l'autre tout en garantissant à la partie d'ancrage à filetage mécanique (4) des libertés de mouvement en translation vertical et en pivotement angulaire par rapport à ladite partie filetée d'ancrage osseux (2) afin de pouvoir pré-positionner axialement avant son immobilisation ladite partie d'ancrage à filetage mécanique (4) par rapport à la position d'ancrage axiale de la partie filetée d'ancrage osseux (2).

2. Vis poly axiale suivant la revendication 1, **caractérisé en ce que** les moyens de retenue (13) sont constitués de deux demi coquilles (13a, 13b) logés à l'intérieur d'une rainure (7) ménagée dans la tête (3) de la partie filetée d'ancrage osseux (2).

3. Vis poly axiale suivant la revendication 2, **caractérisé en ce que** le profil interne des deux demi-coquilles (13a,13b) est de forme complémentaire à celui externe de la tête sphérique (9) de la partie d'ancrage à filetage mécanique (4).

4. Vis poly axiale suivant la revendication 1, **caractérisé en ce que** la partie d'ancrage à filetage mécanique (4) est sécable.

5. Vis poly axiale suivant la revendication 1, **caractérisé en ce que** la partie d'ancrage à filetage mécanique (4) comporte à l'opposé de la tête sphérique (9) une empreinte (10) pour l'introduction d'un outil.

6. Vis poly axiale suivant la revendication 1, **caractérisé en ce que** les moyens de frottement (12) sont constitués d'un fil hélicoïdal (12a) en forme de ressort réalisé dans un matériau à fort coefficient de frottement tel que de l'alliage de titane ou de cobalt.

7. Vis poly axiale suivant la revendication 1, **caractérisé en ce que** le logement (6) aménagé à l'intérieur de la tête (3) de la partie filetée d'ancrage osseux (2) présente un profil sphérique.

8. Vis poly axiale suivant la revendication 1, **caractérisé en ce que** la tête (3) présente sur sa face externe des empreintes (8) permettant la mise en place d'un outil pour l'entrainement en rotation de la partie filetée d'ancrage osseux (2).

9. Vis poly axiale suivant la revendication 1, **caractérisé en ce que** la tête (3) présente sur sa face externe un filetage (3**a**) permettant la mise en place des moyens de retenue (18) assurant que les parties d'ancrage (2, 4) ne puissent se séparer l'une de l'autre tout en garantissant à la partie d'ancrage à filetage mécanique (4) des libertés de mouvement en translation vertical et en pivotement angulaire par rapport à la partie filetée d'ancrage osseux (2)

10. Vis poly axiale suivant la revendication 7, **caractérisé en ce que** le logement (6) aménagé à l'intérieur de la tête (3) de la partie filetée d'ancrage osseux (2) comporte en son milieu une empreinte cylindrique (6**a**) qui est prévue pour recevoir l'une des extrémités du fil hélicoïdal (12**a**) en forme de ressort des moyens de frottement (12).

11. Vis poly axiale suivant la revendication 1, **caractérisé sen ce que** la tête (9) de la partie d'ancrage à filetage mécanique (4) comporte en son milieu une empreinte cylindrique (9**a**) qui est prévue pour recevoir l'une des extrémités du fil hélicoïdal (12**a**) en forme de ressort des moyens de frottement (12).

12. Vis poly axiale suivant les revendications 7 et 10, **caractérisé en ce que** les moyens de retenue sont constitués d'un écrou (18) comportant une face interne sphérique (18**a**) coopérant avec le profil externe sphérique de la tête (9) de la partie d'ancrage à filetage mécanique (4) et une face externe sphérique (18**c**) permettant la réception d'un connecteur de liaison (5) d'un dispositif de fixation rachidien.

## Patentansprüche

1. Polyaxiale Schraube umfassend einen Knochenverankerungsteil mit Gewinde (2) und einen Verankerungsteil mit mechanischem Gewinde (4), jeweils versehen mit einem Kopf (3, 9), wobei diese untereinander mit Haltemitteln (13, 18) verbunden sind, umfassend einerseits Reibungsmittel (12), die zwischen dem Kopf (3) des Knochenverankerungsteils (2) und dem Kopf (9) des Verankerungsteils mit mechanischem Gewinde (4) angeordnet sind, **dadurch gekennzeichnet, dass** sie aus einem schraubenförmigen Draht (12a) in Federform bestehen, der im Inneren eines im Kopf (3) des Knochenverankerungsteils mit Gewinde (2) vorgesehenen Lager (6) platziert ist und auf dem der Kopf (9) mit kugelförmigem Außenprofil des Verankerungsteils mit mechanischem Gewinde (4) aufliegt, und andererseits Haltemittel (13, 18), die sicherstellen, dass die Verankerungsteile (2, 4) sich nicht voneinander trennen können, während sie gewährleisten, dass der Verankerungsteil mit mechanischem Gewinde (4) über Bewegungsfreiheit zur vertikalen Verschiebung und zu einer Winkelschwenkung gegenüber dem Knochenverankerungsteil mit Gewinde (2) verfügt, um den Verankerungsteil mit mechanischem Gewinde (4) gegenüber der axialen Verankerungsposition des Knochenverankerungsteils mit Gewinde (2) vor seiner Immobilisierung axial vorpositionieren zu können.

2. Polyaxiale Schraube gemäß Anspruch 1, **dadurch gekennzeichnet, dass** die Haltemittel (13) aus zwei Halbschalen (13a, 13b) bestehen, die im Inneren einer Nut (7) angeordnet sind, die im Kopf (3) des Knochenverankerungsteils mit Gewinde (2) vorgesehen ist.

3. Polyaxiale Schraube gemäß Anspruch 2, **dadurch gekennzeichnet, dass** das Innenprofil der beiden Halbschalen (13a, 13b) eine komplementäre Form zum Außenprofil des kugelförmigen Kopfes (9) des Verankerungsteils mit mechanischem Gewinde (4) aufweist.

4. Polyaxiale Schraube gemäß Anspruch 1, **dadurch gekennzeichnet, dass** der Verankerungsteil mit mechanischem Gewinde (4) teilbar ist.

5. Polyaxiale Schraube gemäß Anspruch 1, **dadurch gekennzeichnet, dass** der Verankerungsteil mit mechanischem Gewinde (4) gegenüber dem kugelförmigen Kopf (9) eine Vertiefung (10) zur Einführung eines Werkzeugs aufweist.

6. Polyaxiale Schraube gemäß Anspruch 1, **dadurch gekennzeichnet, dass** die Reibungsmittel (12) aus einem schraubenförmigen Draht (12a) in Federform bestehen, der aus einem Material mit hoher Reibungszahl wie beispielsweise einer Titan- oder Kobaltlegierung gefertigt ist.

7. Polyaxiale Schraube gemäß Anspruch 1, **dadurch gekennzeichnet, dass** das Lager (6), das im Inneren des Kopfes (3) des Knochenverankerungsteils mit Gewinde (2) vorgesehen ist, ein kugelförmiges Profil aufweist.

8. Polyaxiale Schraube gemäß Anspruch 1, **dadurch gekennzeichnet, dass** der Kopf (3) auf seiner Außenseite Vertiefungen (8) aufweist, welche das Ansetzen eines Werkzeugs für den Drehantrieb des Knochenverankerungsteils mit Gewinde (2) ermöglichen.

9. Polyaxiale Schraube gemäß Anspruch 1, **dadurch gekennzeichnet, dass** der Kopf (3) auf seiner Außenseite ein Gewinde (3a) aufweist, welches die Anbringung von Haltemitteln (18) ermöglicht, die sicherstellen, dass die Verankerungsteile (2, 4) sich nicht voneinander trennen können, während sie gewährleisten, dass der Verankerungsteil mit mechanischem Gewinde (4) über Bewegungsfreiheit zur vertikalen Verschiebung und zu einer Winkelschwenkung gegenüber dem Knochenverankerungsteil mit Gewinde (2) verfügt.

10. Polyaxiale Schraube gemäß Anspruch 7, **dadurch gekennzeichnet, dass** das Lager (6) im Inneren des Kopfes (3) des Knochenverankerungsteils mit Gewinde (2) in seiner Mitte eine zylinderförmige Vertiefung (6a) aufweist, die vorgesehen ist, um eines der Enden des schraubenförmigen Drahtes (12a) in Federform der Reibungsmittel (12) aufzunehmen.

11. Polyaxiale Schraube gemäß Anspruch 1, **dadurch gekennzeichnet, dass** der Kopf (9) des Verankerungsteils mit mechanischem Gewinde (4) in seiner Mitte eine zylinderförmige Vertiefung (9a) aufweist, die vorgesehen ist, um eines der Enden des schraubenförmigen Drahts (12a) in Federform der Reibungsmittel (12) aufzunehmen.

12. Polyaxiale Schraube gemäß den Ansprüchen 7 und 10, **dadurch gekennzeichnet, dass** die Haltemittel aus einer Mutter (18) bestehen, umfassend eine kugelförmige Innenseite (18a), die mit dem kugelförmigen Außenprofil des Kopfes (9) des Verankerungsteils mit mechanischem Gewinde (4) zusammenpasst, und eine kugelförmige Außenseite (18c), welche die Aufnahme eines Verbindungssteckers (5) einer Wirbelsäulen-Fixiervorrichtung ermöglicht.

## Claims

1. A polyaxial screw comprising a threaded bone anchor part (2) and an anchoring part with a mechanical thread (4) each provided with a head (3, 9) connected to one another by retention means (13, 18), comprising on the one hand friction means (12) arranged between the head (3) of the bone anchor part (2) and the head (9) of the mechanical thread anchoring (4), **characterized in that** the which are constituted by a helicoidal wire (12a) in the form of a spring positioned inside a housing (6) arranged in the head (3) of the thread part of the bone anchor (2) and on which the head (9) comes to be supported which head has an external, spherical profile of the mechanical thread anchor part (4), and comprises on the other hand retention means (13, 18) ensuring that the anchor parts (2, 4) cannon separate from one another while guaranteeing that the mechanical thread anchor part (4) has freedom of movement in vertical translation and in angular pivoting relative to this bone anchor threaded part (2) in order to be able the axially preposition before its immobilization this mechanical thread anchor part (4) relative to the axial anchoring position of the bone anchor threaded part (2).

2. A polyaxial screw according to Claim 1, **characterized in that** the retention means (13) is constituted by two half shells (13a, 13b) housed in a groove (7) arranged in the head (3) of the bone anchor threaded part (2).

3. A polyaxial screw according to Claim 2, **characterized in that** the inner profile of the two half shells (13a, 13b) has a shape complementary to that of the external shape of the spherical head (9) of the mechanical thread anchor part (4).

4. A polyaxial screw according to Claim 1, **characterized in that** the mechanical thread anchor part (4) can be dried.

5. The polyaxial screw according to Claim 1, **characterized in that** the mechanical thread anchor part (4) comprises an impression (10) for the introduction of a tool opposite the spherical head (9).

6. A polyaxial screw according to Claim 1, **characterized in that** the friction means (12) are constituted by a helicoidal wire (12a) in the form of a spring made from a material with a high coefficient of friction such as an alloy of titanium or of cobalt.

7. A polyaxial screw according to Claim 1, **characterized in that** the housing (6) arranged inside the head (3) of the bone anchor threaded part (2) has a spherical profile.

8. A polyaxial screw according to Claim 1, **characterized in that** the head (3) comprises impressions (8) on its external face that allowed the placing of the tool for putting in rotation the bone anchor threaded part (2).

9. A polyaxial screw according to Claim 1, **characterized in that** the head (3) has a thread (3a) on his external face which allows the placing of retention means (18) which ensure that the anchor parts (2, 4) cannot separate from one another while guaranteeing that the mechanical thread anchor part (4) has freedom of movement in vertical translation and in angular pivoting relative to the bone anchor thread part (2).

10. A polyaxial screw according to Claim 7, **characterized in that** the housing (6) arranged inside the head (3) of the bone anchor threaded part (2) comprises a cylindrical impression (6a) in its middle which is provided for receiving one of the ends of the helicoidal wire (12a) in the shape of a spring of the friction means (12).

11. A polyaxial screw according to Claim 1, **characterized in that** the head (9) of the mechanical thread anchor part (4) comprises a cylindrical impression (9a) in its middle which is provided for receiving one of the ends of the helicoidal wire (12a) in the shape of a spring of the friction means (12).

12. A polyaxial screw according to the Claims 7 and 10, **characterized in that** the retention means is constituted by a nut (18) comprising a spherical inner face (18a) cooperating with the spherical outer profile of the head (9) of the mechanical thread anchor part (4) and comprising an outer spherical face (18c) which permits the reception of a connector for linking (5) a spinal fixing device.
